# EUROPEAN PATENT SPECIFICATION

(11) **EP 1 358 187 B1**
(45) Date of publication and mention of the grant of the patent: **10.08.2005**
(21) Application number: 02702326.6
(22) Date of filing: 30.01.2002
(51) Int. Cl.: C07D 471/04, A61K 31/4375, A61P 1/16, A61P 13/12

(54) **NAPHTHYRIDINE DERIVATIVES**
NAPHTHYRIDINDERIVATE
DERIVES DE NAPHTHYRIDINE

(30) Priority: 02.02.2001 GB 0102672
(43) Date of publication of application: 05.11.2003
(73) Proprietor: GLAXO GROUP LIMITED, Greenford, Middlesex UB6 ONN (GB)
(72) Inventor: GELLIBERT, Françoise J., Lab. GlaxoSmithKline, F-919440 Les Ulis (FR); HARTLEY, Charles D., GlaxoSmithKline, Stevenage, Hertfordshire SG1 2NY (GB); MATHEWS, Neil, c/O GlaxoSmithKline, Stevenage, Hertfordshire SG1 2NY (GB); WOOLVEN, James M., GlaxoSmithKline, Stevenage, Hertfordshire SG1 2NY (GB)
(74) Representative: Sewell, Richard Charles
(86) International application number: PCT/EP2002/000939
(87) International publication number: WO 2002/062794

(56) References cited:
- WO-A-00/61576
- WO-A-00/69847

## Description

The present invention relates to novel pyrazole derivatives, processes for the preparation thereof, the use thereof in therapy, particularly in the treatment or prophylaxis of disorders characterised by overexpression of transforming growth factor β (TGF-β), and pharmaceutical compositions for use in such therapy.

TGF-β is a multi-functional cytokine which belongs to the TGF-β superfamily which includes activins/inhibins, bone morphogenetic proteins (BMPs) and TGF- βs. Three isoforms of TGF-β (TGF-β1, TGF-β2, and TGF-β3) have been identified in mammals, each of which is encoded by a distinct gene on different chromosomes (D.A. Lawrence, *Eur. Cytokine. Netw*., 1996, **7(3)**, 363). TGF-β initiates an intracellular signalling pathway which ultimately leads to the expression of genes that regulate cell cycle, control proliferative responses, or relate to extracellular matrix proteins that mediate cell adhesion, migration and intercellular communication. TGF-β has pleitropic effects including modulation of cell growth and differentiation, extracellular matrix formation, hematopoiesis, and immunomodulation (Roberts and Spoon, *Handbook of Experimental Pharmacology,* 1990, **95,** 419-458).

A variety of cell surface proteins and receptors are known to transduce the signals initiated by the binding of the active TGF-β ligand to its receptors. Initiation of the TGF-β signalling pathway results from the binding of the TGF-β ligand to the extracellullar domain of the type II membrane receptor (Massague, *Ann. Rev. Biochem*., 1998, **67**, 753.). The bound type II receptor then recruits type I (Alk5) receptor into a multimeric membrane complex, whereupon active type II receptor kinase phosphorylates and activates type I receptor kinase. The function of the type I receptor kinase is to phosphorylate a receptor-associated co-transcription factor, Smad-2 or Smad-3; thereby releasing it into the cytoplasm where it binds to Smad-4. The PAI-1 gene is activated by TGF-β as a consequence of the abovementioned cellular pathway.

One approach to the treatment and/or prophylaxis of disorders characterised by the overexpression of TGF-β is inhibition of the TGF-β signal transduction. For example, inhibition of the TGF-β type II receptor by overexpression of a dominant negative TGF-β type II receptor has previously been shown to prevent liver fibrosis and dysfunction in rat models (*Proc. Natl. Acad. Sci,* 1999, **96(5),** 2345), and also to prevent progression of established liver fibrosis *(Hepatology,* 2000, **32**, 247).

Pathological overexpression of TGF-β is known to be associated with a number of undesirable effects, leading ultimately to the development of serious pathogenic conditions (G.C. Blobe *et al., N. Engl. J. Med*., 2000, 1350). In particular, pathological overexpression of TGF-β may cause excessive accumulation of extracellular matrix (ECM), inhibition of cell proliferation and immunosuppression. Excessive accumulation of ECM is known to lead to fibrotic diseases such as tumor fibrosis, radiation-induced fibrosis, fibrosis of the liver, kidney, lung, bowel, heart, pancreas, peritoneum or other organs. Fibrosis can lead to pathologic conditions such as cirrhosis, idiopathic pulmonary fibrosis, glomerulosclerosis and hypertrophic scars.

A number of other disease states are known to be associated with variatians in the expression of genes which are controlled by TGF-β including cancer development, abnormal bone function and inflaminatory disorders.

The development of compounds capable of inhibiting the TGF-β intracellular pathway is seen as a desirable way to effect prophylaxis and/or treatment of the above-mentioned conditions. Compounds capable of inhibiting the TGF-β intracellular pathway and/or the expression of TGF-β may be used in the treatment of disorders the symptoms of which often lead to the development of fibrotic conditions. For example, compounds of the present invention may be useful in treating the fibrosis associated with various liver-related conditions such as hepatitis B virus (HBV), hepatitis C virus (HCV), alcohol-induced hepatitis, haemochromatosis and primary biliary cirrhosis.

The compounds of the present invention are pyrazole derivatives. Other pyrazole compounds have previously been described for use in alternative medicinal applications. PCT Patent Applications , WO 96/03385, WO 98/52937, WO 98/52940, WO 98/52941 and WO 00/31063 (Searle & Co) disclose a series of substituted pyrazole compounds and their use in the treatment of p38 kinase mediated disorders. In particular the compounds described are useful in the treatment of inflammation, inflammatory bowel disease, multiple sclerosis and asthma. European Patent Application No. 0 846 687 (Lilly & Co) describes novel substituted pyrazoles useful for the inhibition of sPLA₂ mediated release of fatty acids. Such compounds are useful in the treatment of conditions such as septic shock. EP 0 846 686 (Pfizer Ltd) discloses a series of condensed pyrazole derivatives which act as inhibitors of both Interleukin-1 (IL-1) and tumor necrosis factor (TNF). Such compounds are useful in the treatment of IL-1 and TNF mediated diseases such as chronic inflammatory diseases, specific autoimmune disease and sepsis-induced organ injury. None of the aforementioned patent applications describe the pyrazole compounds of the present invention.

PCT Patent Application WO 00/12947 (Scios Inc.) describes the use of a series of quinazoline derivatives for treating various disorders associated with enhanced activity of kinase p38-α and/or TGF-β. The compounds described therein have been shown to inhibit the activities of both proteins and are therefore particularly useful for the treatment of conditions in which an enhanced activity towards both p38-α and TGF-β is required.

It has now been discovered that certain substituted pyrazole compounds, as described below, are useful in the treatment or prophylaxis of disorders characterised by the overexpression of TGF-β. In particular, compounds of the present invention are TGF-β inhibitors which act at the TGF-β type I (Alk5) receptor level.

According to one aspect of the present invention, we provide compounds of formula (I), 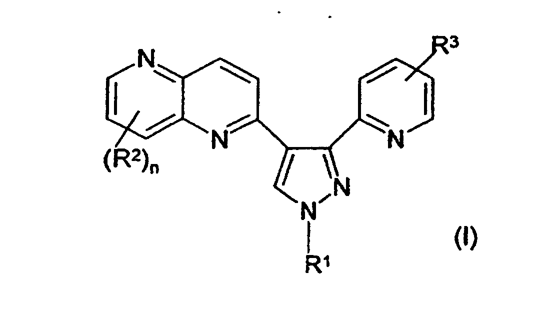 wherein,
R¹ is selected from H, C₁₋₄ alkyl or CH₂CONR⁴R⁵, wherein R⁴ is selected from H or C₁₋₄ alkyl and R⁵ is C₁₋₄ alkyl;
- KHMDS: Potassium *bis*(trimethylsilyl)amide
- MeCN: Acetonitrile
- THF: Tetrahydrofuran

In a further aspect of the present invention, compounds of Formula (I) where R¹ is selected from C₁₋₄ alkyl or CH₂CONR⁴R⁵, wherein R⁴ and R⁵ are hereinbefore defined, may be prepared by N-alkylation of a pyrazole compound of formula (I) obtained from step (vii) above, according to the following general procedure (Scheme 2):
(i) Addition of a suitable N-alkylating reagent such as an alkyl halide, R'X (wherein R¹ is hereinbefore defined) in the presence of a suitable base such as potassium carbonate, sodium hydroxide or potassium hydroxide, preferably in the temperature range 0 to 75°C, more preferably in the temperature range 20 to 60°C, most preferably at room temperature, in the presence of a suitable solvent such as DMF or MeCN, followed by separation of any resulting isomeric mixture by flash chromatography on silica gel.

It will be appreciated that N-alkylation according to step (viii) in Scheme 2 above may result in the formation of structural isomers of a compound of formula (I). Such isomers are afforded by N-alkylation of the tautomeric forms of a compound of formula (I) where R¹ is H, mentioned hereinbefore. The individual isomers and mixtures thereof are included within the scope of the present invention.

6-Methyl-3-aminopyridine (C) may be prepared according to processes known in the art, for example, A.W. Hofmann, *Ber. Dtsch. Ges*., 1881, **14**, 2725.

2-Methyl-1,5-napthyridine (D) may be prepared according to processes known in the art, for example, *Chem. Pharm. Bull.,* 1971, **19(9)**, 1857.

Monosubstituted pyridyl esters, R³(C₅H₃N)CO₂Et (where R³ is hereinbefore defined) as described in step (ii) above may be prepared by processes analogous to those known in the art. For example, where R³ = C(6)-OMe, Finger *et al., J. Org. Chem.,* 1962, **27**, 3965; where R³ = C(3)-OMe, Dejardin *et al., Bull. Chim. Soc. Fr*., 1979, 289; where R³ = C(5)-Br, Chambers and Marfat, *Synth. Commun.,* 1997, 27(3), 515; and where R³ = C(4)-CN, Heinisch and Lotsch, *Heterocycles*, 1987, **26(3)**, 731. mmol) was stirred at room temperature. Glycerol (8.0 ml) was added followed by 3-amino-6-methyl-pyridine (2.79 g, 0.025 mol) and water (14 ml). The resultant mixture was heated at 135 °C with stirring for 18 h. The reaction mixture was allowed to cool to room temperature, basified using 4N sodium hydroxide and the resultant mixture was extracted using ethyl acetate (X4). The extracts were combined and then preadsorbed onto silica gel (20 ml) prior to Biotage chromatography (using a 90 g silica gel cartridge) and eluting with ethyl acetate (neat). Appropriate fractions were combined and then evaporated to give the title compound (2.01 g, 55%) as a light brown crystalline solid.
LC-MS m/z 145 = MH+

### Intermediate 2: 2-[1,5]Naphthyridin-2-yl-1-pyridin-2-yl-ethanone

To a stirred and cooled (-78 °C) solution of 2-methyl-[1,5]naphthyridine, (0.50 g, 3.46 mmol) and ethyl picolinate (0.52 g, 3.47 mmol) in anhydrous THF (30 ml) was added potassium hexamethyldimethylsilazide (0.5M solution in toluene) (13.9 ml, 6.94 mmol) dropwise over 10 minutes. This mixture was stirred at -78 °C for 1 h and then at room temperature for 20 h. Saturated aqueous ammonium chloride (100 ml) was added to the reaction mixture with stirring and the resultant mixture was partitioned between ethyl acetate and water. The aqueous phase was separated off and was extracted with ethyl acetate (X3). The extracts and organic phase were combined, washed with water and finally dried and evaporated to give the title compound (0.86 g) as an orange yellow solid.
[APCl MS] m/z 250 (MH+)

### Intermediate 3: 1-(6-Methyl-pyridin-2-yl)- 2-[1,5]naphthyridin-2-yl-ethanone

2-Methyl-[1,5]naphthyridine (4.34g, 30.1mmol) and methyl-6-methyl picolinate (1.1eq, 5g, 33.11mmol) were coupled as described for intermediate 2 to afford the title compound as an orange solid (6g).
[APCl MS] m/z 264 (MH+)

### Examples

### Example 1: 2-(3-Pyridin-2-yl-1H-pyrazol-4-yl)-1,5-naphthyridine

To a stirred solution of 2-[1,5]naphthyridin-2-yl-1-pyridin-2-yl-ethanone, (0.39 g, 1.56 mmol) in anhydrous DMF (6 ml) was added acetic acid (0.32 ml, 5.61 mmol) followed by dimethylformamide/dimethylacetamide (DMF/DMA) (0.62 ml, 4.68 mmol). This mixture was stirred at room temperature for 1 h and then hydrazine hydrate (1.70 ml, 35.0 mmol) was added. The resultant mixture was heated at 50 °C for 2 h and then allowed to cool to room temperature. The reaction was partitioned between water and ethyl acetate and then the aqueous phase was separated off and was extracted with ethyl acetate (X8). The extracts and organic phase were combined, dried (Na₂SO₄) and evaporated to give crude product as a red-brown gum. This gum was crystallised from acetonitrile to give the title compound (0.31 g, 73%) as a buff coloured solid.
[APCl MS] m/z 274 (MH+)
¹H NMR: (DMSO-d₆): δ 13.6 (1H, V.br.s, NH), 8.92 (1H, dd, CH), 8.58 (1H, br.s, CH), 8.33-8.25 (3H, br.m, 3 X CH), 7.95-7.80 (3H, 2 X m, 3 X CH), 7.74 (1H, dd, CH), 7.40 (1H, m, CH)

### Example 2: 2-[3-(6-Methyl-pyridin-2-yl-1H-pyrazol-4-yl]-[1.5]-naphthyridine

Intermediate 3 (5.66g, 21.52mmol) was reacted with DMF/DMA (3.85g, 32.28mmol) and hydrazine hydrate (7.54g, 150mmol) to afford, after trituration in pentane, the title compound as a yellow solid (2.06g).
m.p: 193°C.
HRMS calcd.Mass for C₁₇H₁₃N₅ : (M+H)⁺ 288.1249, found: 288.1228.

It will be appreciated that a degree of spectral line width broadening is inherent to the ¹H NMR spectra of these types of pyrazole compounds owing to the presence of different tautomeric forms and/or hindered rotation(s) within the molecules.

### Biological Data

The compounds of Examples 1 and 2 were tested *in vitro*, using the biological Assay 1 described below. Both of the compounds tested had an IC₅₀ value of about 0.05 µM in Assay 1.

## Claims

1. A compound of formula (I), 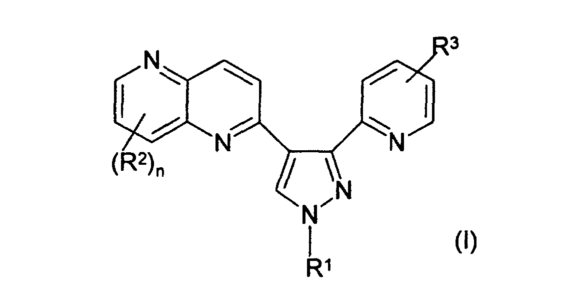 wherein,
R¹ is selected from H, C₁₋₄ alkyl or CH₂CONR⁴R⁵;
n is an integer selected from 0, 1, 2, 3, 4, or 5;
R², which may be the same or different, is selected from halo (such as fluoro, chloro, bromo), -CN, -CF₃, -OCF₃, C₁₋₄ alkyl or C₁₋₄ alkoxy;
R³ is selected from H, halo (such as fluoro, chloro, bromo), -CN, -CF₃, C₁₋₄ alkyl or C₁₋₄ alkoxy;
R⁴ is selected from H or C₁₋₄ alkyl and R⁵ is C₁₋₄ alkyl;
and salts and solvates thereof.

2. A compound of formula (I) as claimed in Claim 1 wherein R¹ is H or C₁₋₄ alkyl.

3. A compound of formula (I) as claimed in Claim 1 or Claim 2, wherein n is 0,1 or 2 and R² is located at the C(2), C(3) or C(4) position of the naphthyridine ring and is selected from halo, -CN, -CF₃, -OCF₃, C₁₋₄ alkyl or C₁₋₄ alkoxy.

4. A compound of formula (I) as claimed in Claim 3, wherein n is 0.

5. A compound of formula (I) as claimed in any one of the preceding claims wherein R³ is located at the C(3) or C(6) position of the pyridine ring and is selected from H, halo, -CN, -CF₃, C₁₋₄ alkyl or C₁₋₄ alkoxy.

6. A compound of formula (I) as claimed in Claim 5, wherein R³ is H or C₁₋₄ alkyl.

7. A compound of formula (I) as claimed in Claim 1 selected from:
2-(3-pyridin-2-yl-1*H*-pyrazol-4-yl)-[1,5]naphthyridine; and
2-[3-(6-methylpyridin-2-yl-1*H*-pyrazol-4-yl]-[1,5]naphthyridine;
and salts and solvates thereof.

8. A pharmaceutical composition comprising at least one compound of formula (I) as claimed in any one of claims 1 to 7, together with a pharmaceutically acceptable diluent or carrier.

9. A compound of formula (I) as claimed in any one of claims 1 to 7, for use as a medicament.

10. The use of a compound of formula (I) as claimed in any one of claims 1 to 7 in the manufacture of a medicament for the treatment and/or prophylaxis of disorders **characterised by** the overexpression of TGF-β.

11. The use of a compound of formula (I) as claimed in any one of claims 1 to 7 in the manufacture of a medicament for the treatment or prophylaxis of a disorder **characterised by** the over expression of TGF-β.

12. The use of a compound of formula (I) as claimed in claim 11, wherein the disorder is selected from fibrosis, especially liver and kidney fibrosis, cancer development, abnormal bone function and inflammatory disorders and scarring.

13. The use of a compound of formula (I) as claimed in claim 10 or claim 11 in combination with an antiviral agent, or in combination with ACE inhibitors or Angiotensin II receptor antagonists.

14. A process for the preparation of a compound of formula (I), 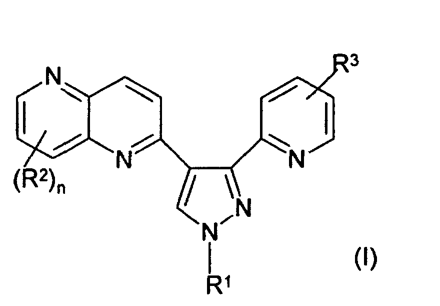 wherein,
R¹ is H; n is an integer selected from 0, 1, 2, 3, 4, or 5; R², which may be the same or different, is selected from halo (such as fluoro, chloro, bromo), -CN, -CF₃, -OCF₃, C₁₋₄ alkyl or C₁₋₄ alkoxy; and R³ is selected from H, halo (such as fluoro, chloro, bromo), -CN, -CF₃, C₁₋₄ alkyl or C₁₋₄ alkoxy;
which process comprises:
a) addition of acetic acid followed by dimethylformamide dimethylacetal to a ketone of formula (E) 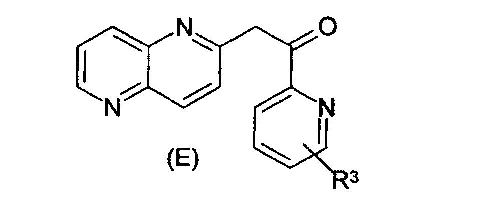 wherein R³ is hereinbefore defined above; and
b) subsequent addition of hydrazine monohydrate, NH₂NH₂.H₂O to the resulting reaction mixture.

15. A process for the preparation of a compound of formula (I), 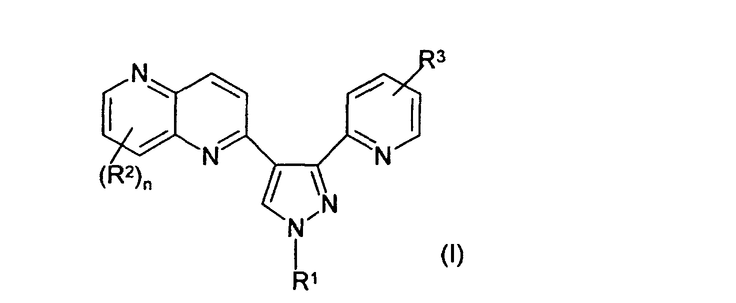 wherein,
R¹ is C₁₋₄ alkyl or CH₂CONR⁴R⁵, wherein R⁴ is selected from H or C₁₋₄ alkyl and R⁵ is C₁₋₄ alkyl; n is an integer selected from 0, 1, 2, 3, 4, or 5; R², which may be the same or different, is selected from halo, -CN, -CF₃, -OCF₃, C₁₋₄ alkyl or C₁₋₄ alkoxy; and R³ is selected from H, halo, -CN, -CF₃, C₁₋₄ alkyl or C₁₋₄ alkoxy;
which process comprises N-alkylation of a compound of formula (I) where R¹ is H, in the presence of a suitable base.

## Patentansprüche

1. Verbindung der Formel (I), 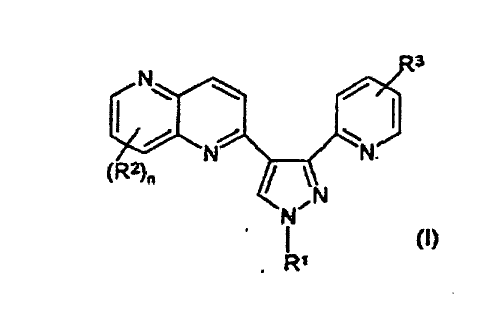 wobei
R¹ aus H, C₁₋₄-Alkyl oder CH₂CONR⁴R⁵ ausgewählt ist;
n eine ganze Zahl, ausgewählt aus 0, 1, 2, 3, 4 oder 5, ist;
R², das gleich oder verschieden sein kann, aus Halogen (wie Fluor, Chlor, Brom), -CN, -CF₃, -OCF₃, C₁₋₄-Alkyl oder C₁₋₄-Alkoxy ausgewählt ist;
R³ aus H, Halogen (wie Fluor, Chlor, Brom), -CN, -CF₃, C₁₋₄-Alkyl oder C₁₋₄-Alkoxy ausgewählt ist;
R⁴ aus H oder C₁₋₄-Alkyl ausgewählt ist und R⁵ C₁₋₄-Alkyl ist;
und Salze und Solvate davon.

2. Verbindung der Formel (I) gemäß Anspruch 1, wobei R¹ H oder C₁₋₄-Alkyl ist.

3. Verbindung der Formel (I) gemäß Anspruch 1 oder Anspruch 2, wobei n 0, 1 oder 2 ist und R² sich an der C(2)-, C(3)- oder C(4)-Position des Naphthyridinrings befindet und aus Halogen, -CN, -CF₃, -OCF₃, C₁₋₄-Alkyl oder C₁₋₄-Alkoxy ausgewählt ist.

4. Verbindung der Formel (I) gemäß Anspruch 3, wobei n 0 ist.

5. Verbindung der Formel (I) gemäß einem der vorstehenden Ansprüche, wobei R³ sich an der C(3)- oder C(6)-Position des Pyridinrings befindet und aus H, Halogen, -CN, -CF₃, C₁₋₄-Alkyl oder C₁₋₄-Alkoxy ausgewählt ist.

6. Verbindung der Formel (I) gemäß Anspruch 5, wobei R³ H oder C₁₋₄-Alkyl ist.

7. Verbindung der Formel (I) gemäß Anspruch 1, ausgewählt aus:
2-(3-Pyridin-2-yl-1*H*-pyrazol-4-yl)-[1,5]naphthyridin; und
2-[3-(6-Methylpyridin-2-yl-1*H*-pyrazol-4-yl]-[1,5]naphthyridin;
und Salzen und Solvaten davon.

8. Arzneimittel, umfassend mindestens eine Verbindung der Formel (I) gemäß einem der Ansprüche 1 bis 7, zusammen mit einem pharmazeutisch verträglichen Verdünnungsmittel oder Träger.

9. Verbindung der Formel (I) gemäß einem der Ansprüche 1 bis 7 zur Verwendung als Medikament.

10. Verwendung einer Verbindung der Formel (I) gemäß einem der Ansprüche 1 bis 7 zur Herstellung eines Medikaments zur Behandlung und/oder Prophylaxe von Störungen, die durch die Überexpression von TGF-β **gekennzeichnet** sind.

11. Verwendung einer Verbindung der Formel (I) gemäß einem der Ansprüche 1 bis 7 zur Herstellung eines Medikaments zur Behandlung oder Prophylaxe einer Störung, die durch Überexpression von TGF-β **gekennzeichnet** ist.

12. Verwendung einer Verbindung der Formel (I) gemäß Anspruch 11, wobei die Störung aus Fibrose, insbesondere Leber- und Nierenfibrose, Krebsentwicklung, abnormaler Knochenfunktion und entzündlichen Störungen und Narbenbildung ausgewählt ist.

13. Verwendung einer Verbindung der Formel (I) gemäß Anspruch 10 oder Anspruch 11 in Verbindung mit einem Antivirenmittel oder in Verbindung mit ACE-Inhibitoren oder Angiotensin-II-Rezeptorantagonisten.

14. Verfahren zur Herstellung einer Verbindung der Formel (I), 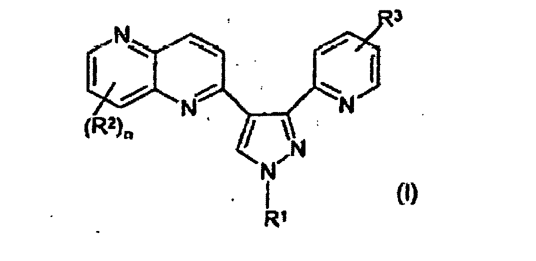 wobei
R¹ H ist; n eine ganze Zahl, ausgewählt aus 0, 1, 2, 3, 4 oder 5, ist; R², das gleich oder verschieden sein kann, aus Halogen (wie Fluor, Chlor, Brom), -CN, -CF₃, -OCF₃, C₁₋₄-Alkyl oder C₁₋₄-Alkoxy ausgewählt ist; und R³ aus H, Halogen (wie Fluor, Chlor, Brom), -CN, -GF³, C₁₋₄-Alkyl oder C₁₋₄-Alkoxy ausgewählt ist;
wobei das Verfahren umfasst:
a) Zugabe von Essigsäure gefolgt von Dimethylformamiddimethylacetal zu einem Keton der Formel (E) 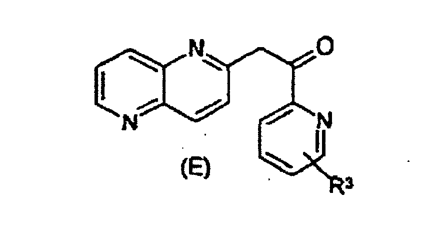 wobei R³ hier vorstehend definiert ist; und
b) nachfolgende Zugabe von Hydrazinmonohydrat, NH₂NH₂·H₂O, zu dem resultierenden Reaktionsgemisch.

15. Verfahren zur Herstellung einer Verbindung der Formel (I) 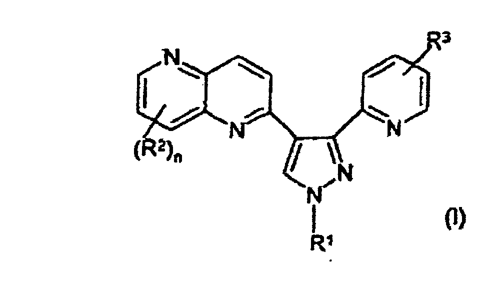 wobei
R¹ C₁₋₄-Alkyl oder CH₂CONR⁴R⁵ ist, wobei R⁴ aus H oder C₁₋₄-Alkyl ausgewählt ist und R⁵ C₁₋₄-Alkyl ist; n eine ganze Zahl, ausgewählt aus 0, 1, 2, 3, 4 oder 5, ist; R², das gleich oder verschieden sein kann, aus Halogen, -CN, -CF₃, -OCF₃, C₁₋₄-Alkyl oder C₁₋₄-Alkoxy ausgewählt ist; und R³ aus H, Halogen, -CN, -CF₃, C₁₋₄-Alkyl oder C₁₋₄-Alkoxy ausgewählt ist;
wobei das Verfahren N-Alkylierung einer Verbindung der Formel (I), wobei R¹ H ist, in Gegenwart einer geeigneten Base umfasst.

## Revendications

1. Composé de formule (I), 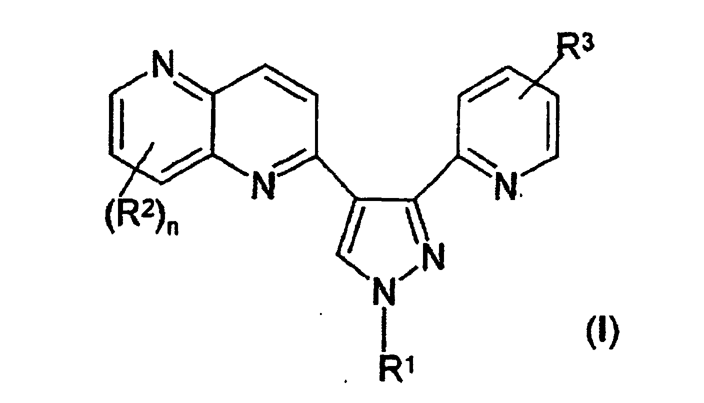 dans laquelle,
R¹ est choisi entre H, un groupe alkyle en C₁ à C₄ et un groupe CH₂CONR⁴R⁵ ;
n représente un nombre entier choisi entre 0, 1, 2, 3, 4 et 5 ;
les groupes R², qui peuvent être identiques ou différents, sont choisis entre des groupes halogéno (par exemple fluoro, chloro, bromo), -CN, -CF₃, -OCF₃, alkyle en C₁ à C₄ et alkoxy en C₁ à C₄ ;
R³ est choisi entre H, des groupes halogéno (par exemple fluoro, chloro, bromo), -CN, -CF₃, alkyle en C₁ à C₄ et alkoxy en C₁ à C₄ ;
R⁴ est choisi entre H et un groupe alkyle en C₁ à C₄ et R⁵ représente un groupe alkyle en C₁ à C₄ ;
et ses sels et produits de solvatation.

2. Composé de formule (I) suivant la revendication 1, dans lequel R¹ représente H ou un groupe alkyle en C₁ à C₄.

3. Composé de formule (I) suivant la revendication 1 ou la revendication 2, dans lequel n est égal à 0, 1 ou 2 et R² est situé en position C(2), C(3) ou C(4) du noyau naphtyridine et est choisi entre des groupes halogéno, -CN, -CF₃, -OCF₃, alkyle en C₁ à C₄ et alkoxy en C₁ à C₄.

4. Composé de formule (I) suivant la revendication 3, dans lequel n est égal à 0.

5. Composé de formule (I) suivant l'une quelconque des revendications précédentes, dans lequel R³ est situé en position C(3) ou C(6) du noyau pyridine et est choisi entre H, des groupes halogéno, -CN, -CF₃, alkyle en C₁ à C₄ et alkoxy en C₁ à C₄.

6. Composé de formule (I) suivant la revendication 5, dans lequel R³ représente H ou un groupe alkyle en C₁ à C₄.

7. Composé de formule (I) suivant la revendication 1, choisi entre :
la 2-(3-pyridin-2-yl-1*H*-pyrazol-4-yl)-[1,5]naphtyridine ; et
la 2-[3-(6-méthylpyridin-2-yl-1H-pyrazol-4-yl]-[1,5]-naphtyridine ;
et leurs sels et produits de solvatation.

8. Composition pharmaceutique comprenant au moins un composé de formule (I) suivant l'une quelconque des revendications 1 à 7, conjointement avec un diluant ou support pharmaceutiquement efficace.

9. Composé de formule (I) suivant l'une quelconque des revendications 1 à 7, destiné à être utilisé comme médicament.

10. Utilisation d'un composé de formule (I) suivant l'une quelconque des revendications 1 à 7 dans la production d'un médicament destiné au traitement et/ou à la prophylaxie de troubles **caractérisés par** la surexpression de TGF-β.

11. Utilisation d'un composé de formule (I) suivant l'une quelconque des revendications 1 à 7 dans la production d'un médicament destiné au traitement ou à la prophylaxie d'une affection **caractérisée par** la surexpression du TGF-β.

12. Utilisation d'un composé de formule (I) suivant la revendication 11, dans laquelle l'affection est choisie entre la fibrose, notamment la fibrose hépatique et la fibrose rénale, le développement d'un cancer, une fonction osseuse anormale et des troubles inflammatoires et la cicatrisation.

13. Utilisation d'un composé de formule (I) suivant la revendication 10 ou la revendication 11, en association avec un agent antiviral, ou en association avec des inhibiteurs de ACE ou des antagonistes du récepteur d'angiotensine II.

14. Procédé pour la préparation d'un composé de formule (I), 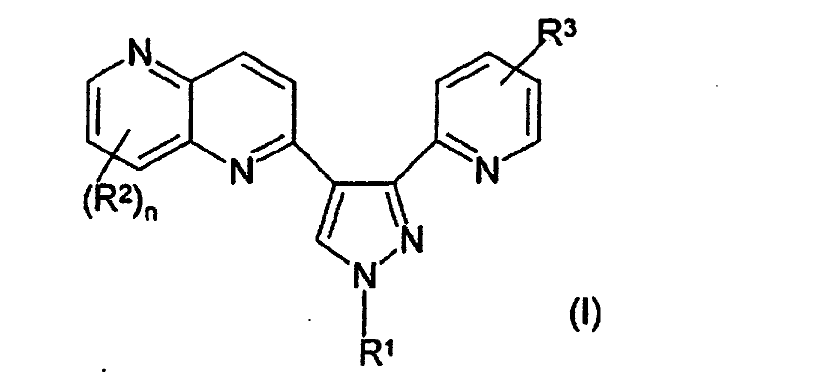 dans laquelle,
R¹ représente H ; n représente un nombre entier choisi entre 0, 1, 2, 3, 4 et 5 ; les groupes R², qui peuvent être identiques ou différents, sont choisis entre des groupes halogéno (par exemple fluoro, chloro, bromo), -CN, -CF₃, -OCF₃, alkyle en C₁ à C₄ et alkoxy en C₁ à C₄ ; et R³ est choisi entre H, des groupes halogéno (par exemple fluoro, chloro, bromo), -CN, -CF₃, alkyle en C₁ à C₄ et alkoxy en C₁ à C_{4 ;}
procédé qui comprend :
a) l'addition d'acide acétique puis d'acétaldiméthylique de diméthylformamide à une cétone de formule (E) 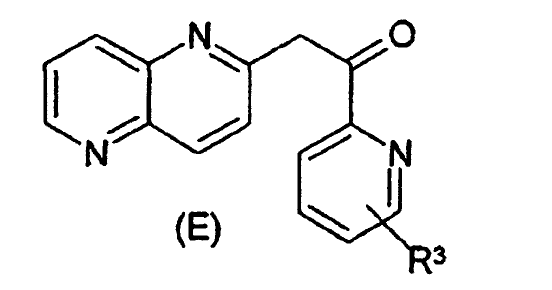 dans laquelle R³ répond à la définition précitée ; et
b) ensuite l'addition de monohydrate d'hydrazine, NH₂NH₂.H₂O, au mélange résultant.

15. Procédé pour la préparation d'un composé de formule (I) 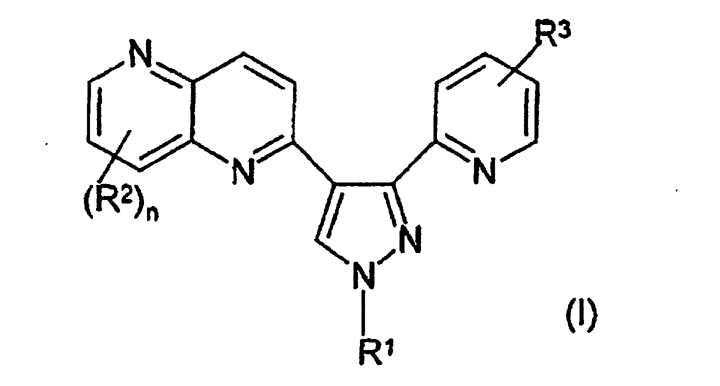 dans laquelle,
R¹ représente un groupe alkyle en C¹ à C⁴ ou CH₂CONR⁴R⁵, dans lequel R⁴ est choisi entre H et un groupe alkyle en C₁ à C₄ et R⁵ représente un groupe alkyle en C₁ à C₄ ; n représente un nombre entier choisi entre 0, 1, 2, 3, 4 et 5 ; les groupes R², qui peuvent être identiques ou différents, sont choisis entre des groupes halogéno, CN, -CF₃, -OCF₃, alkyle en C₁ à C₄ et alkoxy en C₁ à C₄ ; et R³ est choisi entre H, des groupes halogéno, CN, -CF₃, alkyle en C₁ à C₄ et alkoxy en C₁ à C₄ ;
procédé qui comprend la N-alkylation d'un composé de formule (I) dans laquelle R¹ représente H, en présence d'une base convenable.
